(19) Europäisches Patentamt
European Patent Office
Office européen des brevets

(11) Veröffentlichungsnummer : **0 186 836 B2**

(12) # NEUE EUROPÄISCHE PATENTSCHRIFT

(45) Veröffentlichungstag der neuen Patentschrift : **18.03.92 Patentblatt 92/12**

(51) Int. Cl.$^5$ : **B01D 61/44**

(21) Anmeldenummer : **85115968.1**

(22) Anmeldetag : **13.12.85**

(54) **Verfahren zur Aufkonzentrierung wässriger Lösungen von organischen Verbindungen, die Salze enthalten, unter gleichzeitiger Verringerung des Salzgehaltes.**

(30) Priorität : **21.12.84 DE 3446695**

(43) Veröffentlichungstag der Anmeldung : **09.07.86 Patentblatt 86/28**

(45) Bekanntmachung des Hinweises auf die Patenterteilung : **03.08.88 Patentblatt 88/31**

(45) Bekanntmachung des Hinweises auf die Entscheidung über den Einspruch : **18.03.92 Patentblatt 92/12**

(84) Benannte Vertragsstaaten : **BE CH DE FR GB IT LI NL**

(56) Entgegenhaltungen : **EP-A- 0 029 960**

(56) Entgegenhaltungen :
**EP-A- 0 167 107**
**DE-A- 2 805 891**
**Journal of Membrane Science, Bd. 14, Nr. 3, Juli 1983, Amsterdam, Ch.A. Kruissink "The effect of electro-osmotic water transport", S. 331-368**
**Angewandte Chemie, 94/9, 1982, Weinheim, W. Pusch/A. Walch "Synthetische Membranen", S. 670-695**

(73) Patentinhaber : **BASF Aktiengesellschaft
Carl-Bosch-Strasse 38
W-6700 Ludwigshafen (DE)**

(72) Erfinder : **Puetter, Hermann, Dr.
Haardter Strasse 1
W-6730 Neustadt (DE)**
Erfinder : **Roske, Eckhard
Hillensheimer Strasse 4
W-6700 Ludwigshafen (DE)**

EP 0 186 836 B2

## Beschreibung

Diese Erfindung betrifft ein Verfahren zur Aufkonzentrierung wäßriger Lösungen von ionischen organischen Verbindungen, die Salze enthalten, unter gleichzeitiger Verringerung des Salzgehaltes dieser Lösungen durch Elektrodialyse.

Die Gewinnung salzarmer wäßriger Lösungen organischer Verbindungen ist von erheblicher technischer Bedeutung. Zur Verringerung des Salzgehaltes von wäßrigen Lösungen organischer Verbindungen werden häufig Membranverfahren, wie die Ultrafiltration oder Reverse Osmose eingesetzt. Die Ultrafiltration gestattet die gleichzeitige Entfernung von Lösungsmitteln und niedermolekularen Salzen von organischen Verbindungen, ist aber auf hochmolekulare Produkte beschränkt. Ihre optimalen Trenneigenschaften entfaltet sie im Bereich der Makromoleküle (Partikelgröße größer als $10^{-2}$ µm) während organische Verbindungen mit einem Molekulargewicht im Bereich von $10^3$ oder darunter (Partikelgröße kleiner als $10^{-3}$ µm) durch Ultrafiltration nicht von Salzen getrennt werden können.

Die Reverse Osmose ermöglicht die Aufkonzentrierung von Lösungen organischer Verbindungen durch Abtrennung von Lösungsmitteln, insbesondere von Wasser. Sie kann aber im allgemeinen Salze nicht von anderen Stoffen abtrennen, so daß sich bei der Aufkonzentrierung des Wertproduktes störende ionogene Verunreinigungen ansammeln.

Es hat nicht an Versuchen gefehlt, Verfahren im Grenzbereich zwischen Ultrafiltration und Reverse Osmose zu entwickeln, um organische Verbindungen von Salzen abzutrennen. Bei diesen Verfahren wird zwar eine gewisse Aufkonzentration erzielt, sie sind im allgemeinen jedoch auf Moleküle vom Molekulargewicht >1000, meist >5000 beschränkt. Erschwerend kommt hinzu, daß es eine scharfe Trenngrenze nicht gibt. So lassen sich selbst mit den besten Membranen nur Stoffe voneinander trennen, die sich im Molekulargewicht um den Faktor 2 oder höher voneinander unterscheiden.

Für die Abtrennung von Salzen aus wäßrigen Lösungen hat man auch schon die Elektrodialyse herangezogen, z.B. für die Entsalzung von Brackwasser und zur Herstellung von Speisesalz. In beiden Fällen ist das Ziel das gleiche: Abtrennung von Salz (NaCl) mit möglichst geringem Transport von Wasser. Auf gute Leistungen in dieser Richtung sind deshalb auch alle modernen Ionenaustauschermembranen optimiert. Zur Steigerung der Stromausbeute und Entsalzungsrate bei der Elektrodialyse werden zwischen Diluat und Konzentrat größere Konzentrationsunterschiede vermieden, da der Salzgehalt im Diluat bei hohen Salzgehalten im Konzentrat durch Rückdiffusion nicht unter einen Grenzwert von etwa 0,1 % abgesenkt werden kann. Bei hohen Salzkonzentrationen im Konzentrat (>1 molar) bricht außerdem die Permselektivität der Membranen zusammen, sie liegt in verdünnten Lösungen bei 85 bis 95 %.

Die Elektrodialyse wird z.B. in H. Strathmann "Trennung von molekularen Mischungen mit Hilfe synthetischer Membranen", Steinkopf, Darmstadt, 1979, S. 76 bis 86 und in D.S. Flett "Ion Exchange Membranes", Ellis Horwood, Chichester 1983, S. 179 bis 191 beschrieben.

Es wurde nun gefunden, daß man wäßrige Lösungen von ionischen organischen Verbindungen, die Salze enthalten, unter gleichzeitiger Verringerung des Salzgehaltes aufkonzentrieren kann, wenn man die salzhaltige wäßrige Lösung der organischen Verbindung elektrodialysiert und dabei als Konzentrat eine mindestens 1-molare wäßrige Salzlösung verwendet.

Ausgangslösungen für das neue Verfahren sind durch Salze oder den freien Säuren, die diesen Salzen zugrundeliegen, verunreinigte wäßrige Lösungen von farblosen oder farbigen organischen Verbindungen. Sie können als organische Verbindungen wasserlösliche organische Verbindungen der verschiedensten Art enthalten. Das sind z.B. ionische Verbindungen, wie organische Säuren z.B. Carbonsäuren, Sulfonsäuren, Phosphonsäuren, Metallkomplexverbindungen, Heterocyclen, Ammoniumverbindungen, wie quaternäre Verbindungen, Phosphoniumsalze, Betaine, wie Aminocarbonsäuren, Aminosulfonsäuren oder heterocyclische Betaine oder elektroneutrale organische Verbindungen, wie Kohlenhydrate,Ether, Nitrile, Amine, Azoverbindungen, Heterocyclen oder wasserlösliche Oligomere oder Polymere.

Die untere Molekulargewichtsgrenze, bis zu der sich die organischen Verbindungen von den Salzen abtrennen lassen, hängt davon ab, ob die Verbindungen elektroneutralen oder ionischen Charakter haben. Bei Neutralstoffen liegt sie etwa bei 60, bei ionogenen Stoffen etwa bei 120. Unter diesen Werten kann man zwar ebenfalls Trennwirkungen erzielen, jedoch muß dann mit größeren Verlusten an der organischen Verbindung gerechnet werden. Nach oben hin ist das Molekulargewicht der organischen Verbindung nicht begrenzt, wenn nur eine gewisse Wasserlöslichkeit gegeben ist. Bevorzugt ist bei ionogenen monomeren organischen Verbindungen ein Molekulargewichtsbereich von 200 bis 2 000. Es lassen sich z.B. auch wasserlösliche Oligomere oder Polymere mit Molekulargewichten von 2000 bis 2 Millionen einsetzen.

Die wäßrigen Lösungen der ionischen organischen Verbindungen enthalten als Salze z.B. die Halogenide, Nitrate, Sulfate, Carbonate, Sulfide, Sulfite, Formiate, Acetate, Phosphate der Alkalimetalle, der Erdalkalimetalle, des Ammoniums oder in Wasser löslichen Salze von Schwermetallen, wie Kupfer, Zink, Silber, Zinn und

Eisen. Nach dem Verfahren der Erfindung wird auch ein etwaiger Gehalt an den freien Säuren, die diesen Salzen zugrundeliegen, wie Salzsäure, Schwefelsäure, Kohlensäure, Essigsäure, Ameisensäure, Salpetersäure oder Phosphorsäure verringert.

Für das neue Verfahren lassen sich wäßrige Lösungen einsetzen, die z.B. bis zu 50 Gew.-% an der organischen Verbindung enthalten und einen Salzgehalt bis zu 30 Gew.-% aufweisen.

Als Konzentrat wird für das neue Verfahren eine mindestens 1-molare wäßrige Salzlösung verwendet. Als Salze kommen z.B. wasserlösliche Halogenide, Sulfide, Sulfite, Sulfate, Carbonate, Formiate oder Acetate in Betracht, vorzugsweise solche des Ammoniums oder der Alkali- oder Erdalkalimetalle. Besonders geeignet sind z.B. NaCl, KCl, LiCl, $NH_4Cl$, $Na_2SO_4$, $Li_2SO_4$ und $K_2SO_4$, von denen die Sulfate bevorzugt sind. Insbesondere verwendet man mindestens 3 molare wäßrige Lösungen der Chloride oder Bromide der Alkalimetalle oder eine bei Raumtemperatur gesättigte Lösung der genannten Salze.

Man elektrodialysiert auf an sich bekannte Weise in Elektrodialyse-Apparaten, wie man sie z.B. für die Entsalzung von Brackwasser, Fruchtsäften und Molke verwendet, bei Temperaturen bis 100°C, vorzugsweise bei 15 bis 80°C. Die Stromdichte beträgt zwischen 10 und 0,01 $A/dm^2$, vorzugsweise zwischen 5 bis 0.05 $A/dm^2$. Die Zellenspannung beträgt pro Teilzelle 0,5 bis 2 V, wobei eine Teilzelle aus einer Diluatkammer und einer Konzentratkammer besteht.

Nach dem neuen Verfahren wird eine erhebliche Aufkonzentrierung salzhaltiger Lösungen von ionischen organischen Verbindungen bei gleichzeitiger Verringerung des Salzgehaltes ohne größere Einbußen an Stromausbeute erzielt. Dieses vorteilhafte Ergebnis war nicht zu erwarten, weil man bei den hohen Salzkonzentrationen im Konzentrat damit rechnen mußte, daß keine oder eine nur sehr geringe Entsalzung der Diluate erzielt werden kann. Überraschenderweise haben aber die hohen Salzkonzentrationen im Konzentrat auf den Entsalzungseffekt nicht den erwarteten negativen Einfluß. So können die Salzkonzentrationen im Konzentrat um mehr als doppelt so hoch sein, als es für eine optimale Permselektivität notwendig wäre. Sie können sogar um mehr als das Hundertfache höher sein als die Salzkonzentrationen, die verfahrensgemäß im Diluat erreicht werden. Gleichermaßen überraschend ist, daß selbst bei eingesetzten Lösungen mit hohen Gehalten an organischen Verbindungen, wie solchen mit Konzentrationen von über 10 und sogar über 30 % nennenswerte Verluste an der organischen Verbindung nicht beobachtet werden. Erstaunlich ist auch, daß sich sogar wäßrige Lösungen von organischen Verbindungen mit Molekulargewichten unter 200 vorteilhaft aufkonzentrieren lassen und daß bei Verwendung von Lösungen, die stark ionische organische Verbindungen mit Molekulargewichten von >200 enthalten, der Substanzverlust nur sehr gering ist. Es war auch keineswegs vorhersehbar, dass das erzielte Ausmass der Aufkonzentrierung so erheblich über die durch die normale Elektroosmose erwartete Entfernung von Wasser hinausgehen würde.

Die erfindungsgemässe Elektrodialyse ist z.B. der Ultrafiltration auch bei der Entsalzung und Aufkonzentrierung von Lösungen höhermolekularer Verbindungen überlegen. Das ist insbesondere dann von Bedeutung, wenn ein Produkt mit hohem Salzanteil auf einen sehr niedrigen Salzanteil gebracht werden soll. Beispielsweise lässt sich eine 30%ige wässrige Lösung des polymeren A mit einem Salzgehalt (NaCl) von 15% durch Ultrafiltration in einem Durchlauf auf einen Gehalt von 60% A und 7,5% NaCl bringen. 50% der Ausgangslösung gehen dabei (im Idealfall) als 15%ige NaCl-Lösung ins Permeat. Durch anschliessendes Verdünnen mit Wasser auf das alte Volumen und erneute Ultrafiltration sinkt der Salzgehalt erneut um die Hälfte und damit auf 3,75%. Insgesamt muss der Prozess 5mal durchgeführt werden, um einen Restsalzgehalt von <0,5% zu erzielen (Entsalzungsgrad ≧95%). Die gleiche Wirkung kann mit der erfindungsgemässen Elektrodialyse in einem Durchgang erzielt werden. Das erfindungsgemässe Verfahren stellt somit auch eine kostengünstige Alternative zur Ultrafiltration dar, wenn auch Salze abgetrennt werden sollen.

Man kann bei dem erfindungsgemässen Verfahren auch einen Teil der Gleichstromenergie unter Verzicht auf hohe Stromausbeuten dazu nutzen, die Isolierung des Wertproduktes zu erleichtern, Während es in den meisten Fällen ausreichend ist, die Lösung um den Faktor 1,2 bis 2 aufzukonzentrieren und den Salzgehalt der Lösung zu senken, kann es von Interesse sein, z.B. wenn das Wertprodukt als Trockensubstanz isoliert werden soll, die Lösung noch weiter aufzukonzentrieren, beispielsweise bis um den Faktor ≧ 3. Dabei sinkt zwar oft der Salzgehalt der Lösung nicht weiter, der Salzgehalt bezogen auf das Wertprodukt wird aber noch weiter abgesenkt. Ein besonderer Vorteil dieser Ausführungsform der Erfindung besteht zusätzlich noch darin, dass Verdampfungskapazität eingespart wird. Dies erhöht die Flexibilität des Verfahrens und senkt die thermische Belastung empfindlicher Produkte. Insbesondere beim Betrieb kleinerer Chargen führt die erfindungsgemässe Verfahrensweise auch zum Absenken der Energiekosten. Diese besondere Ausführungsform des Verfahrens nutzt den überraschenden Effekt des erfindungsgemässen Verfahrens, der darin besteht, dass eine über die normale Elektroosmose hinausgehende Entfernung von Wasser eintritt.

Das erfindungsgemässe Verfahren erweitert auch die Anwendbarkeit der herkömmlichen Elektrodialyseverfahren, bei denen es oft technisch schwierig ist, Ausgangslösungen zu verarbeiten, die infolge eines hohen Salzgehaltes Niederschläge enthalten (Aussalzeffekt) oder stärker viskos sind. Man kann die Ausgangslösung

nun zunächst verdünnen und nach der erfindungsgemässen Elektrodialyse auf die gewünschte Ausgangskonzentration anreichern. Auf diese Weise umgeht man Verstopfungen der Zelle, Belegung der Membranen und sichert durch ausreichende Beströmung eine hohe Entsalzungsgeschwindigkeit in der Zelle. Im allgemeinen werden auch unerwünschte Polarisationserscheinungen (diese gefürchteten pH-Verschiebungen in Diluat und Konzentrat führen zur frühzeitigen Zerstörung der Membranen) durch die erfindungsgemässe Verfahrensweise unterdrückt.

Die in den Beispielen genannten Prozente sind Gewichtsprozente. Unter « Aufkonzentrierungsfaktor » wird der Quotient aus der Gewichtsmenge des eingesetzten Diluats und der Gewichtsmenge des erhaltenen Diluats und unter Entsalzungsgrad der Quotient aus den Gewichtsmengen an Salz im eingesetzten und erhaltenen Diluat verstanden.

*Beispiel 1*

a) Apparatur:

Die Elektrodialysezelle, deren Schema aus der Figur ersichtlich ist, ist über Polyethylenschläuche mit drei voneinander getrennten Kreisläufen verbunden, die die Zelle aus Vorratsbehältern mit dem Konzentrat (1), dem Diluat (2) und dem Elektrolyten (3) versorgen. Die Zelle besitzt zwei Platinelektroden (4) von je 0,35 $dm^2$. Die Elektrodenräume (5) sind von den angrenzenden Konzentraträumen (6) durch Kationenaustauschermembranen (7), die im Handel unter der Bezeichnung ®Nafion erhältlich sind, abgetrennt. Zwischen den Elektrodenräumen befinden sich sechs Konzentraträume (6) und 5 Diluaträume (8) in alternierender Anordnung. Die Räume sind voneinander abwechselnd durch Kationenaustauschermembranen (9), die im Handel unter der Bezeichnung ®Selemion CMV bzw. Anionenaustauschermembranen (10), die im Handel unter der Bezeichnung ®Selemion AMV erhältlich sind, getrennt. Die Membranen haben eine aktive Fläche von 0,37 $dm^2$ und sind durch PVC-Rahmen so befestigt, dass sie einen Abstand voneinander von 2 mm aufweisen. An den Rahmen sind auch die Zulauf- und Ablaufanordnungen angebracht, über welche die Kreisläufe angeschlossen sind. Jeder der drei Kreisläufe ist mit einer Magnetkreiselpumpe und einem Wärmetauscher versehen. Die Apparatur ist mit Gleichstromversorgung, Temperatur-, pH- und Leitfähigkeitsmessung sowie mit Stickstoff-Spülung ausgerüstet.

b) Durchführung der Elektrodialysen:

Die drei Kreisläufe der Apparatur wurden mit den folgenden Lösungen beschickt:

| | |
|---|---|
| Elektrolyt: | 1100 g 5%ige wässrige $NaSO_4$-Lösung |
| Konzentrat: | 4000 g gesättigte wässrige NaCl-Lösung mit NaCl-Bodensatz |
| Diluat: | 1500 g 5%ige wässrige Lösung des Farbstoffes der Formel |

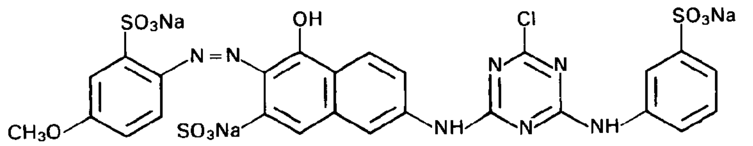

(Mg: 803,5)

Die Farbstofflösung hatte einen Chloridgehalt von 0,58%.

Die Elektrodialyse wurde bei 30°C und 25 V Klemmspannung durchgeführt. Dabei wurden die Lösungen umgepumpt. Die Anfangsstärke von ca. 1,2 A sank mit dem Fortschreiten der Entsalzung, die analytisch überprüft wurde ($Cl^{\ominus}$-Bestimmung), allmählich ab. Die Elektrodialyse wurde nach 18 h Laufzeit abgebrochen. Es wurde als Diluat eine aufkonzentrierte wässrige Lösung des Farbstoffes erhalten, die einen Chloridgehalt von 0,11% aufwies. Dabei wurde folgendes Ergebnis erzielt:

| | |
|---|---|
| Aufkonzentrierungsfaktor (Diluat): | 1,88 |
| Entsalzungsgrad (Diluat): | ca. 89% |
| Stromausbeute: | 10,6% |
| Materialausbeute: | >99% |

*Beispiel 2* (Vergleichsbeispiel )

Man elektrodialysierte wie im Beispiel 1 beschrieben, wobei man jedoch als Konzentrat 1000 g einer 0,5%igen wässrigen NaCl-Lösung und als Diluat 1500 g einer 10%igen wässrigen Lösung des Farbstoffes mit einem Chloridgehalt von 0,31% verwendete. Es wurde als Diluat eine weitgehend salzfreie Farbstofflösung erhalten, bei der aber so gut wie keine Aufkonzentrierung der Lösung eingetreten war. Erzieltes Ergebnis:

| | |
|---|---|
| Aufkonzentrierungsfaktor: | 1,04 |
| Entsalzungsgrad: | >99% |
| Stromausbeute: | ca. 46% |
| Materialausbeite: | >99% |

*Beispiel 3*

1500 g einer 5%igen wässrigen Lösung des Farbstoffes der Formel

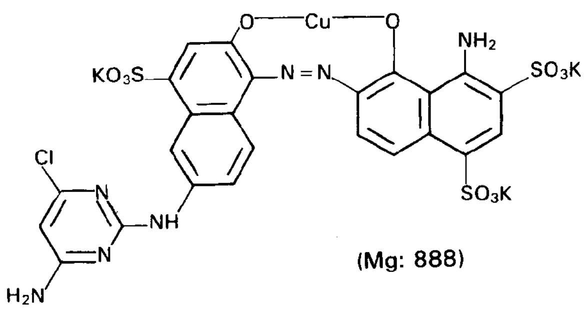

(Mg: 888)

mit einem Chloridgehalt von 0,55% wurde analog Beispiel 1 elektrodialysiert, wobei jedoch als Konzentrat 4000 g einer gesättigten wässrigen Kaliumchloridlösung, die KCl-Bodensalz enthielt, eingesetzt wurde. Nach einer Laufzeit von 43 h wurde die Elektrodialyse abgebrochen. Es wurde eine aufkonzentrierte wässrige Lösung des Farbstoffes mit einem Chloridgehalt von 0,56% erhalten. Ergebnis:

| | |
|---|---|
| Aufkonzentrierungsfaktor: | 7,5 |
| Entsalzungsgrad: | ca. 86% |
| Stromausbeute: | ca. 4% |
| Materialausbeite: | >99% |

*Beispiel 4*

Die drei Kreisläufe der in Beispiel 1 verwendeten Apparatur wurden mit den folgenden Lösungen beschickt:
Diluat: 1500 g einer 10%igen wässrigen Lösung des Farbstoffes der Formel

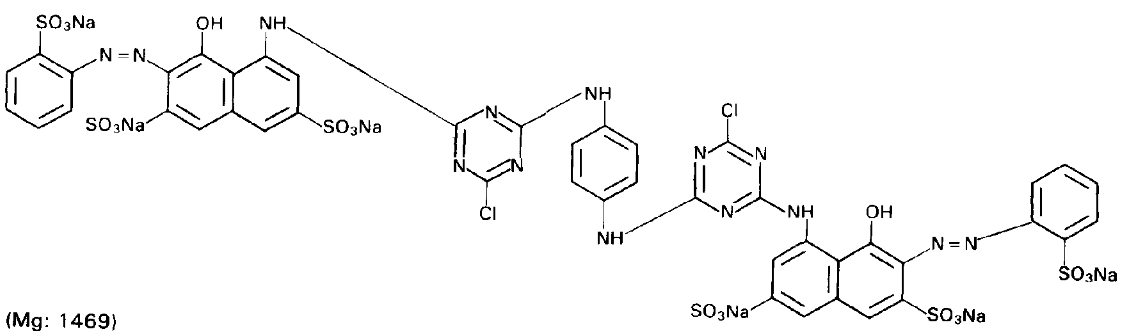

(Mg: 1469)

Die Farbstofflösung hatte einen Chloridgehalt (NaCl, KCl) von 1,99%
Konzentrat: 4000 g gesättigte, wässrige NaCl-Lösung mit Bodensatz
Elektrolyt: 1000 g 5%ige wässrige $NA_2SO_4$-Lösung.

Die Elektrodialyse wurde bei einer Zellspannung von 25 V und einer Stromstärke von anfangs 1,6 A begonnen. Nach einer Betriebszeit von 27 h betrug die Stromstärke 0,52 A. Die Elektrodialyse wurde abgebrochen.

Die Diluatmenge war auf 456,9 g abgesunken. Der Chloridgehalt des Diluats betrug 0,24%. Der Feststoffgehalt war auf ca. 30% angestiegen.

| Ergebnis: | |
|---|---|
| Aufkonzentrierungsfaktor: | 3,28 |
| Entsalzungsgrad: | 96% |
| Stromausbeute: | 25% |
| Materialausbeute: | >99% |

*Beispiel 5*

Die drei Kreisläufe der in Beispiel 1 beschriebenen Apparatur wurden mit den folgenden Lösungen beschickt:

| | |
|---|---|
| Diluat: | 2000 g einer wässrigen Sarkosinlösung, die 2,8 Mol/kg Sarkosin (MG: 89) enthielt und einen Chloridgehalt (NaCl) von 9,78% aufwies |
| Konzentrat: | 2000 g einer bei 25°C gesättigten wässrigen NaCl-Lösung |
| Elektrolyt: | 1500 g 5%ige wässrige $Na_2SO_4$-Lösung. |

Man elektrodialysierte 30 h bei einer Zellspannung von ≦30 V und einer Stromstärke von ≦2 A, wobei alle 3 h das Konzentrat gewechselt wurde. Am Ende war die Diluatmenge auf 1100 g abgesunken. Das Diluat hatte einen Gehalt an Sarkosin von 4,20 Mol/kg und einen Chloridgehalt von 0,02%.

| Ergebnis: | |
|---|---|
| Aufkonzentrierungsfaktor: | 1,82 |
| Entsalzungsgrad: | >99% |
| Stromausbeute: | 60% |
| Materialausbeute: | 83% |

*Beispiel 6*

Die drei Kreisläufe der in Beispiel 1 beschriebenen Apparatur wurden mit den folgenden Lösungen beschickt:

| | |
|---|---|
| Diluat: | 2000 g einer wässrigen γ-Aminobuttersäurelösung, mit einem Gehalt an γ-Aminobuttersäure (MG: 103) von 2,38 Mol/kg und einem Chloridgehalt (NaCl) von 8,61% |
| Konzentrat: | 2000 g einer bei 25°C gesättigten wässrigen NaCl-Lösung |
| Elektrolyt: | 1500 g 5%ige wässrige $Na_2SO_4$-Lösung. |

Es wurde 18 h bei einer Zellspannung von ≦20 V und einer Stromstärke von ≦2 A elektrodialysiert, wobei alle 3 h das Konzentrat gewechselt wurde. Am Ende war die Diluatmenge auf 1233 g abgesunken. Das Diluat hatte einen Gehalt anγ-Aminobuttersäure von 3,62 Mol/kg und einen Chloridgehalt von 0,02%.

| Ergebnis: | |
|---|---|
| Aufkonzentrierungsfaktor: | 1,62 |
| Entsalzungsgrad: | >99% |
| Stromausbeute: | 68% |
| Materialausbeute: | 94% |

*Beispiel 7*

Die drei Kreisläufe der in Beispiel 1 beschriebenen Apparatur wurden mit den folgenden Lösungen beschickt:

| | |
|---|---|
| Diluat: | 2000 g einer 5, 75%igen wässrigen Arabinoselösung, einem Chloridgehalt (NaCl) von 7,02% |
| Konzentrat: | 2 500 einer gesättigten wässrigen NaCl-Lösung |
| Elektrolyt: | 1500 g 5%ige wässrige $Na_2SO_4$-Lösung |

Es wurde 15 h bei einer Zellspannung von ≦34 V und einer Stromstärke von ≦2 A elektrodialysiert, wobei alle 3 h das Konzentrat gewechselt wurde. Am Ende war die Diluatmenge auf 996 g abgesunken. Das Diluat hatte einen Gehalt an Arabinose von 10,49% und einen Chloridgehalt von 0,01%.

| Ergebnis: | |
|---|---|
| Aufkonzentrierungsfaktor: | 2,01 |
| Entsalzungsgrad: | >99% |
| Stromausbeute: | 77% |
| Materialausbeute: | 91% |

*Beispiel 8* (Vergleichsbeispiel)

Es wurde wie in Beispiel 7 beschrieben elektrodialysiert, wobei jedoch als Diluat 2000 g einer 5,94%igen wässrigen Arabinoselösung mit einem Chloridgehalt (NaCl) von 7,02% und als Konzentrat eine 0,5%ige wässrige NaCl-Lösung verwendet wurde. Das Konzentrat wurde auch hier alle 3 h gewechselt (in technischen Anlagen wird das Konzentrat in der Regel kontinuierlich über einen Überlauf ausgetragen und mit Wasser aufgefüllt, um die Salzkonzentration in Konzentrat und Diluat nicht zu stark auseinandergehen zu lassen). Am Ende betrug die Endkonzentration an Chlorid im Konzentrat 0,38 %. Die Diluatmenge war auf 1344 g abgesunken. Das Diluat hatte einen Gehalt an Arabinose von 8,17% und einen Chloridgehalt von 0,14%.

| Ergebnis: | |
|---|---|
| Aufkonzentrierungsfaktor: | 1,49 |
| Entsalzungsgrad: | >99% |
| Stromausbeute: | 89% |
| Materialausbeute: | 92% |

Bei einem Vergleich mit Beispiel 7 ersieht man, dass bei gleicher Versuchsdauer eine nahezu gleiche Materialausbeute und eine geringfügig schlechtere Stromausbeute erhalten wird, während nach Beispiel 7 ein um 35% erhöhter Aufkonzentrierungsfaktor erzielt wird.

*Beispiel 9*

Es wurde eine ähnliche Apparatur wie in Beispiel 1 beschrieben verwendet, die anstelle von 6 Konzentraträumen 20 Konzentraträume und anstelle von 5 Diluaträumen 19 Diluaträume aufwies. Die aktive Membran- und Elektrodenfläche betrug 2 $dm^2$. Als Kationenaustauschermembranen wurden die im Handel unter der Bezeichnung ®Neosepta, CH-45 T-Membranen und als Anionenaustauschermembranen ®Neosepta, ACH-45 T-Membranen verwendet. Die drei Kreisläufe wurden mit folgenden Lösungen beschickt:

Diluat: 9985 g einer wässrigen γ-Aminobuttersäurelösung mit einem Gehalt an γ-Aminobuttersäure von 0,49 Mol/kg und einem Sulfatgehalt ($Na_2SO_4$) von 8,4%

Konzentrat: 3992 g einer 15%igen wässrigen $Na_2SO_4$-Lösung.

Bei einer Zellenspannung von ≦30 V und einer Stromstärke von ≦6 A wurde 6,5 h elektrodialysiert. Am Ende war die Diluatmenge auf 5461 g abgesunken, der Gehalt an Aminobuttersäure auf 0,70 Mol/kg angestiegen, der Sulfatgehalt betrug 0,7%.

| Ergebnis: | |
|---|---|
| Aufkonzentrationsfaktor: | 1,83 |
| Entsalzungsgrad: | 95% |
| Stromausbeute: | 86% |
| Materialausbeute: | 79% |

*Beispiel 10*

Die drei Kreisläufe der in Beispiel 1 verwendeten Apparatur wurden mit den folgenden Lösungen beschickt:

Diluat: 1500 g einer 5%igen, wässrigen Polydimethyldiallylammoniumchloridlösung mit einem NaCl-Gehalt von 2%
Konzentrat: 3000 g einer gesättigten wässrigen NaCl-Lösung mit NaCl-Bodensatz
Elektrolyt: 1000 g einer 5%igen wässrigen $Na_2SO_4$-Lösung.

Bei 20°C, einer Zellspannung von 25 V und einer anfänglichen Stromstärke von 1,13 A wurde in 27,5 h die Diluatmenge auf 704 g gesenkt und das NaCl weitgehend abgereichert.

| Ergebnis: | |
|---|---|
| Aufkonzentrationsfaktor: | 2,13 |
| Entsalzungsgrad: | 97,1% |
| Stromausbeute: | 16,7% |
| Materialausbeute: | 98,3% |

*Beispiel 11*

Die drei Kreisläufe der in Beispiel 1 verwendeten Apparatur wurden mit den folgenden Lösungen beschickt:

Diluat: 1500 g einer 5%igen, wässrigen Polydimethyldiallylammoniumchloridlösung mit einem NaCl-Gehalt von 2%
Konzentrat: 3000 g einer gesättigten wässrigen NaCl-Lösung mit NaCl-Bodensatz
Elektrolyt: 1000 g einer 5%igen wässrigen $Na_2SO_4$-Lösung.

Bei 60°C, einer Zellspannung von 25 V und 2 A Anfangsstromstärke sank bei weitgehender NaCl-Abreicherung die Diluatmenge in 16 h auf 315 g ab.

| Ergebnis: | |
|---|---|
| Aufkonzentrationsfaktor: | 4,75 |
| Entsalzungsgrad: | 97,4% |
| Stromausbeute: | 13,0% |
| Materialausbeute: | 95,7% |

## Patentansprüche

1. Verfahren zur Aufkonzentrierung wäßriger Lösungen von ionischen organischen Verbindungen, die Salze enthalten, unter gleichzeitiger Verringerung des Salzgehaltes dieser Lösungen, dadurch gekennzeichnet, daß man die salzhaltige wäßrige Lösung der organischen Verbindung elektrodialysiert, wobei man als Konzentrat eine mindestens 1-molare wäßrige Salzlösung verwendet.

2. Verfahren zur Aufkonzentrierung wäßriger Lösungen von elektroneutralen organischen Verbindungen, die Salze enthalten, unter gleichzeitiger Verringerung des Salzgehaltes dieser Lösungen, dadurch gekennzeichnet, daß man die salzhaltige wäßrige Lösung der organischen Verbindung elektrodialysiert, wobei man als Konzentrat eine mindestens 3-molare wäßrige Lösung der Chloride oder Bromide der Alkalimetalle verwendet.

3. Verfahren nach Anspruch 1, dadurch gekennzeichnet, daß man als Konzentrat eine mindestens 1-molare wäßrige Lösung eines Alkalisulfates verwendet.

4. Verfahren nach Anspruch 1, dadurch gekennzeichnet, daß man als Konzentrat eine mindestens 3-molare wäßrige Lösung der Chloride oder Bromide der Alkalimetalle verwendet.

5. Verfahren nach Anspruch 1, dadurch gekennzeichnet, daß man als Konzentrat eine bei Raumtemperatur gesättigte Salzlösung verwendet.

6. Verfahren nach Anspruch 1, dadurch gekennzeichnet, daß man als Salze für das Konzentrat wasserlösliche Halogenide, Sulfide, Sulfite, Sulfate, Carbonate, Formiate oder Acetate verwendet.

7. Verfahren nach Anspruch 1, dadurch gekennzeichnet, daß man als Salze für das Konzentrat LiCl, KCl, NaCl, $NH_4Cl$, $Li_2SO_4$, $K_2SO_4$ oder $Na_2SO_4$ verwendet.

8. Verfahren nach den Ansprüchen 1 und 2, dadurch gekennzeichnet, daß man bei Temperaturen bis 80°C elektrodialysiert.

## Claims

1. A process for concentrating a salt-containing aqueous solution of an ionic organic compound, with simultaneous reduction of the salt content of this solution, wherein the salt-containing aqueous solution of the organic compound is subjected to electrodialysis, the concentrate used being an aqueous salt solution which is not less than 1 molar.

2. A process for concentrating a salt-containing aqueous solution of an electroneutral organic compound, with simultaneous reduction of the salt content of this solution, wherein the salt-containing aqueous solution of the organic compound is subjected to electrodialysis, the concentrate used being an aqueous alkali metal chloride or bromide solution which is not less than 3 molar.

3. A process as claimed in claim 1, wherein the concentrate used is an aqueous alkali metal sulfate solution which is not less than 1 molar.

4. A process as claimed in claim 1, wherein the concentrate used is an aqueous alkali metal chloride or bromide solution which is not less than 3 molar.

5. A process as claimed in claim 1, wherein the concentrate used is a salt solution which is saturated at room temperature.

6. A process as claimed in claim 1, wherein the salt used for the concentrate is a water-soluble halide, sulfide, sulfite, sulfate, carbonate, formate or acetate.

7. A process as claimed in claim 1, wherein the salt used for the concentrate is LiCl, KCl, NaCl, $NH_4Cl$, $Li_2SO_4$, $K_2SO_4$ or $Na_2SO_4$.

8. A process as claimed in claim 1 or 2, wherein electrodialysis is carried out at no higher than 80°C.

## Revendications

1. Procédé de concentration de solutions aqueuses de composés organiques ioniques, qui contiennent des sels, avec réduction simultanée de la teneur en sels de ces solutions, caractérisé en ce que l'on soumet la solution aqueuse du composé organique contenant des sels à une électrodialyse, et on utilise, à titre de concentrat, une solution aqueuse de sels au moins 1 molaire.

2. Procédé de concentration de solutions aqueuses de composés organiques électroneutres, qui contiennent des sels, avec réduction simultanée de la teneur en sels de ces solutions, caractérisé en ce qu'on soumet la solution aqueuse du composé organique contenant des sels à une électrodialyse, et on utilise à titre de concentrat une solution aqueuse au moins 3 fois molaire des chlorures ou des bromures de métaux alcalins.

3. Procédé suivant la revendication 1, caractérisé en ce que l'on utilise, à titre de concentrat, une solution aqueuse d'un sulfate de métal alcalin au moins 1 fois molaire.

4. Procédé suivant la revendication 1, caractérisé en ce que l'on utilise, à titre de concentrat, une solution aqueuse des chlorures ou des bromures des métaux alcalins, au moins 3 fois molaire.

5. Procédé suivant la revendication 1, caractérisé en ce que l'on utilise, à titre de concentrat, une solution de sels, saturée à la température ambiante.

6. Procédé suivant la revendication 1, caractérisé en ce que l'on utilise à titre de sels pour le concentrat, des halogénures, sulfures, sulfites, sulfates, carbonates, formiates ou acétates solubles dans l'eau.

7. Procédé suivant la revendication 1, caractérisé en ce que l'on utilise, à titre de sels pour le concentrat, les composés suivants : LiCl, KCl, NaCl, $NH_4Cl$, $Li_2SO_4$, $K_2SO_4$ ou $Na_2SO_4$.

8. Procédé suivant la revendication 1, caractérisé en ce que l'on procède à l'électrodialyse à des températures allant jusqu'à 80°C.

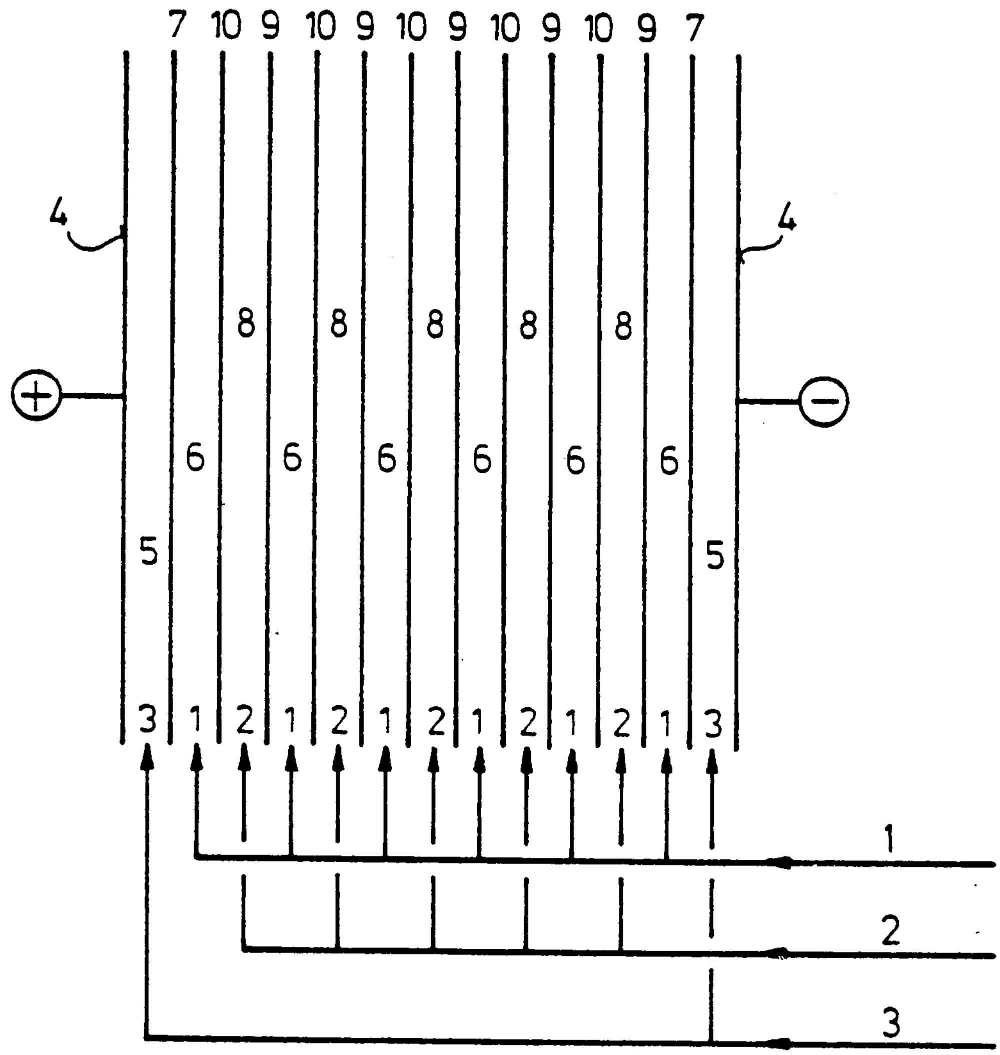
7 10 9 10 9 10 9 10 9 10 9 7
4
4
8 8 8 8 8
6 6 6 6 6 6
5
5
3 1 2 1 2 1 2 1 2 1 2 1 3
1
2
3